(19) Europäisches Patentamt
European Patent Office
Office européen des brevets

(11) Publication number: **0 179 444 B1**

(12) **EUROPEAN PATENT SPECIFICATION**

(45) Date of publication of patent specification: **12.06.91** (51) Int. Cl.5: **A61K 9/50**, A61K 49/02

(21) Application number: **85113386.8**

(22) Date of filing: **22.10.85**

(54) **Use of a micellular particle composition encapsulating a chemotherapeutic agent for intravenous targeting to tumors.**

(30) Priority: **22.10.84 US 663550**
**22.10.84 US 663503**

(43) Date of publication of application:
**30.04.86 Bulletin 86/18**

(45) Publication of the grant of the patent:
**12.06.91 Bulletin 91/24**

(84) Designated Contracting States:
**AT BE CH DE FR GB IT LI LU NL SE**

(56) References cited:
**EP-A- 0 028 917**

**SCIENCE, vol. 207, 18th January 1980, pages 309-311; M.R. MAUK et al.: "Visicle targeting: Timed release and specificity for leukocytes in mice by subcutaneous injection"**

**CHEMICAL ABSTRACTS, vol. 92, 1980, page 339, abstract no. 169129j, Columbus, Ohio, US; R.S. CHAWLA et al.: "The effect of liposomal charge on drug toxicity and efflux"**

(73) Proprietor: **VESTAR, INC.**
**650 Cliffside Drive**
**San Dimas California 91773(US)**

(72) Inventor: **Presant, Cary Arnett**
**2345 Cumberland Road**
**San Marino, Calif. 91108(US)**
Inventor: **Proffitt, Richard Thomas**
**11 North Altura Road**
**Arcadia, Calif. 91006(US)**
Inventor: **Teplitz, Raymond Leo**
**801 Eigth Street**
**Davis Calif. 95616(US)**
Inventor: **Williams, Lawrence Ernest**
**130 Greenfield Place**
**Arcadia Calif. 91106(US)**
Inventor: **Tin, George Wing-Yiu**
**431 East Sandra**
**Arcadia, Calif. 91006(US)**

(74) Representative: **Eitle, Werner, Dipl.-Ing. et al**
**Hoffmann, Eitle & Partner Patentanwälte Arabellastrasse 4**
**W-8000 München 81(DE)**

EP 0 179 444 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 99, no. 6, August 1983, pages 306-307, abstract no. 43393s, Columbus, Ohio, US; P. MACHY et al.: "Small liposomes are better than large liposomes for specific drug delivery in vitro"

CHEMICAL ABSTRACTS, vol. 93, no. 26, December 1980, page 382, abstract no. 245393d, Columbus, Ohio, US; G. GREGORIADIS et al.: "The phospolipid component of small unilamellar liposomes controls the rate of clearence of entrapped solutes from the circulation"

CHEMICAL ABSTRACTS, vol. 99, 1983, page 314, abstract no. 43459t, Columbus, Ohio, US; G.L. WHITE et al.: "The influence of cholesterol on the stability of liposomes containing methotrexate"

## Description

This invention relates to the use of a micellular particle composition encapsulating a chemotherapeutic agent for targeting to tumors. More particularly, the invention relates to the use of neutral or charged phospholipid micellular particle compositions containing a chemotherapeutic agent for treating tumors in a body.

Before various abnormalities such as tumors in a patient's body can be diagnosed and treated, it is often necessary to locate the abnormalities. This is particularly true of such abnormalities as malignant tumors since the treatment of such tumors is often on a localized basis. For example, the location of malignant tumor cells has to be identified so that a chemotherapeutic agent can be directed to such cells to eliminate the tumor.

Numerous attempts have been made over many years to identify specific locations, such as tumor locations, in a patient's body by simple techniques. For example, it would be desirable for diagnostic purposes to identify the location of cancer cells in a patient's body by a simple method involving the introduction of selected mobile particles to the patient's body and by the movement of such particles to the cancer cells. It would also be desirable to treat such cancer cells by introducing chemotherapeutic agents into the patient's body and having such agents move to such specific locations to combat the cancer cells at such locations. Except for recent advances in the use of monoclonal antibodies, simple and reliable methods for targeting specific locations such as tumors for diagnosis, and methods for successfully delivering chemotherapeutic agents to the tumors in a patient's body for treatment, have not been developed.

Placing a chemotherapeutic drug in the body orally, subcutaneously or intravenously can result in harm to the normal cells in the body which take up the drug and a worsening in the patient's condition, without achieving the desired reduction in tumor cell activity. In the past, this toxicity to normal cells in the patient's body has been a major disadvantage in the treatment of tumors with chemotherapeutic agents. The lack of efficacy of such chemotherapy is also attributable to the failure of the freely circulating drug to localize within tumor cells before it is excreted or taken up by other cells in the body.

Prior attempts to improve treatment of tumors by chemotherapeutic agents have included encapsulation of such agents within biodegradable phospholipid micellular particles in the form of vesicles or liposomes. Encapsulation is thought to reduce the potential toxicity from the circulating drugs. Researchers have also sought to utilize such encapsulation to selectively target tumors within a body for delivery of chemotherapeutics. However, until the present invention efforts to reliably place imaging agents or drug-encapsulating particles within tumor cells have not been demonstrated.

Because solid tumors and their metastases are located in extravascular tissues, to accomplish targeting of intravenously injected imaging or chemotherapeutic agents to the tumor cells, the agents must leave the normal circulation by crossing blood vessel membranes to enter the extravascular tissues. This movement is known as "extravasation". Normally, small substances such as small molecular weight proteins and membrane-soluble molecules can cross tumor capillary walls by a process known as passive diffusion. However, passive diffusion was thought not to allow sufficient accumulation of larger particles carrying drugs to reach therapeutic levels within the vicinity of the tumor. H.I. Peterson, Vascular and Extravascular Spaces in Tumors: Tumor Vascular Permeability, Chapter III, Tumor Blood Circulation, H.I. Peterson, Ed. (1979).

Progress in targeting specific locations, such as tumor locations, with chemotherapeutic drugs encapsulated in particles such as vesicles has been hampered by the inability to achieve movement of encapsulated drug across blood vessel membranes and to detect such movement. In the usual case, large structures such as drug encapsulating vesicles cannot escape from blood vessels such as capillaries, and thus remain in the circulation. However, an examination of the structure of the vascular morphology of a tumor reveals that the various blood vessels associated with tumors, in particular capillaries, exhibit alterations in their structure as a result of tumor cell growth patterns. Studies of tumor capillary permeability suggest that these morphologic variations in the capillaries allow some substances to cross the capillary membrane. Such variations include defects in the vascular endothelium from poor cell differentiation, and breaks in vascular walls as a result of invading tumor cells. Examples of tumor-modified capillaries include vessels with interrupted endothelial lining and vessels with fenestrated endothelium. (H.I. Peterson, supra).

Notwithstanding such knowledge of tumor vascular morphology, researchers such as Peterson have concluded that transport of large molecules or materials across the tumor capillary wall occurs as a result of passive diffusion only and that "concentrations of active drugs sufficient for therapeutic effect are difficult to reach." (H.I. Peterson, supra, at page 83).

Prior to such morphologic studies, early research on the problem of extravasation suggested that vesicles might undergo "transcapillary passage" across the capillary membranes and on into tumor cells. (G. Gregoriadis, Liposomes In Biological Systems, Gregoriadis, Ed., Ch. 2, (1980)). However, available data indicated that the vesicles were unstable in vivo and that the radiolabel may have leaked, apparently prompting alternative theories such as prolonged circulation of vesicles and the release of drugs from such vesicles at a slower rate, and interaction of the liposomes with the capillary walls without actually crossing the wall surface, which presumably resulted in the drugs being detected within tumors. (Id.) Other researchers simply have concluded that the vesicles do not penetrate vascular walls after intravenous administration. (B. Ryman et al., Biol. Cell, Vol. 47, pp. 71-80 (1983); G. Poste, Biol. Cell, Vol 47, pp. 19-38 (1983); G. Poste et al., Novel Approaches to Cancer Chemotherapy, Academic Press, p. 166-230 (1984); G. Poste, Receptor Mediated Targeting of Drugs, Plenum Press, p. 427-473 (1985)).

The publication by M.R. Mauk et al in Science, Vol. 207 (1980) p. 309-311, relates to subcutaneous injections of vesicles in mice and deals with the time release and specificity for leukocytes. This article does not refer to the intravenous administration of vesicles, and consequently does not relate to circulation of such vesicles in the bloodstream to tumor sites and their accumulation within tumor cells.

EP-28 917 describes vesicles accumulating within the lymphatic system or the lung, spleen or liver. This document does not deal with the targeting of tumor sites for a successfull treatment of the tumor.

Chemical Abstracts, Vol. 99, No. 9, August 1983, p. 306-307, Abstract No. 43393s by Machy et al describes drug transfer into target cells in vitro. Thus, this abstract does not describe injecting liposomes into the bloodstream and circulating intact to accumulate within tumor cells.

Thus, although the prior art has recognized the necessity that vesicles carrying therapeutic drugs must cross vascular barriers to reach tumor cells, the experience of the art has taught that intravenous administration of micellular particles such as phospholipid vesicles is not effective to deliver encapsulated drugs to extravascular tumor cells.

The object of the invention is to enhance extravasation of encapsulated therapeutic agents to tumor cells within the body to deliver said chemotherapeutic agents to the cells of such tumors.

Above object is solved according to the invention by the use of a micellular particle composition, said particles having a chemotherapeutic agent encapsulated therein and comprising a chemically pure (>98% pure)phospholipid and zero to fifty percent cholesterol by weight of phospholipid, said particles having a size less than 200 nm, for the preparation of an intravenously administrable pharmaceutical composition useful for the delivery of said particle composition and chemotherapeutic agent into tumor cells.

The micellular particle compositions to be used according to the invention comprise biodegradable micellular particles in the form of unilamellar phospholipid vesicles. Pure (more than approximately 98% pure) neutral phospholipid molecules containing hydrocarbon chains having at least 18 carbon atoms, such as distearoylphosphatidylcholine (DSPC), are incorporated into the vesicles.

The chemotherapeutic agent may be associated with the phospholipid molecules or internal contents of the particles to treat the tumors. A particularly useful chemotherapeutic agent is methotrexate.

When the phospholipid vesicles as described herein are introduced into the bloodstream of a patient, they move intact to locate in specific locations in the patient's body, such as locations where cancerous growth appears. This cancerous growth at specific locations can then be treated.

To enhance the movement of the phospholipid vesicles containing imaging agents or chemotherapeutic agents vesicles to the tumors in the patient's body, a first group of phospholipid vesicles having positively charged molecules extending externally from the particles may be introduced into the patient's bloodstream to block uptake by phagocytic cells such as those in the liver, spleen and other tissues in the patient's body comprising the reticuloendothelial system. The extended, positively charged molecules bound to such phospholipid vesicles may be lipid soluble molecules containing a protruding amino group, for example, aminosaccharide derivatives of cholesterol, such as 6-aminomannose derivative of cholesterol. Concurrently, or after a suitable period of time, such as approximately one hour, a second group of small (less than 200nm)phospholipid vesicles may be introduced into the patient's bloodstream to place intact this second group of vesicles in the specific locations such as tumors in the patient's body. Such phospholipid vesicles preferably are neutral, and may include cholesterol.

Figure 1 is a table illustrating the targeting of phospholipid vesicles to tumors in a body.

Figure 2 is a table illustrating the targeting of reticuloendothelial cells in the liver and spleen by phospholipid vesicles which may be used as a blocking agent.

Figure 3 is a table illustrating the targeting of phospholipid vesicles to tumors in the body after the blocking of the reticuloendothelial cells in the liver and spleen.

Figure 4 is a chart illustrating the time course of clearance of radiolabeled phospholipid vesicles in the blood and accumulation in the tumor.

Figure 5 is a graph indicating the percentage of intact labelled phospholipid vesicles remaining in blood and tumor as determined by gamma ray perturbed angular correlation (PAC) spectroscopy.

Figure 6 is a table illustrating the blood distribution of neutral labeled vesicles incorporating Indium-111 chelated to ehtylene-diamine tetracetic acid (EDTA) or C-14 Dipalmitoyl phosphatidylcholine as compared to the In-111-EDTA in various tissues.

Figure 7 is a table illustrating the blood distribution of labeled phospholipid vesicles in tissues of mice bearing 10 different tumors.

Figure 8 is a series of autoradiographs depicting tumor cells containing labeled vesicles.

Figure 9 is a table illustrating the enhanced delivery of a chemotherapeutic agent to a tumor in the body by the use of phospholipid vesicles.

Figure 10 is a table showing enhanced delivery of a vesicle encapsulated chemotherapeutic to tumor.

As used herein, "micellular particles" and "micelles" refer to water-soluble-particles which result from spontaneous aggregations of amphiphilic molecules. Amphiphilic molecules contain hydrophobic and hydrophilic portions. In this invention, preferred amphiphiles are biological lipids. Such micelles can be in the form of small spheres, ellipsoids or long cylinders, and can also consist of bilayers with two parallel layers of amphiphilic molecules. Such bilayered micelles usually take the shape of unilamellar spherical vesicles with an internal aqueous compartment, and are also known as "liposomes."

Methods for forming these vesicles are, by now, well known in the art. Typically, they are prepared from phospholipids, for example, distearoylphosphatidylcholine by sonication, and may include other materials such as neutral lipids, for example, cholesterol, and also surface modifiers such as positively or negatively charged compounds, saccharides, antibodies and other functional ligands which have groups that can anchor the molecule in the bilayer of the vesicle. We have found that by incorporating certain phospholipid molecules, a vesicle is obtained which is stable in vivo. It is known that phase transition points are a function of hydrocarbon chain length,(C. Tanford, The Hydrophobic Effect, 2nd. Ed. (1980)). Certain phospholipid molecules exhibit phase transitions at relatively high temperatures (greater than 37° C), and we have found that use of these phospholipids in the compositions described herein provide vesicles with improved stability in vivo.

The stability of the phospholipid micellular particles may be further enhanced by incorporating cholesterol. A stable vesicle may be obtained by incorporating 0-50% cholesterol by weight of phospholipid into the vesicles.

Vesicle Preparation.

Small unilamellar vesicles (SUV) with the ionophore A23187 were prepared from distearoylphosphatidylcholine (DSPC), cholesterol (Ch), dicetyl phosphate (DP), stearylamine (SA) and the 6-aminomannose (AM), and 6-aminomannitol (AML) derivatives of cholesterol, according to previous methods. (Mauk and Gamble, Anal. Bioc., 94, 302-307 (1979)),

Briefly, chloroform solutions of 10 mg lipid with the following molar ratios: DSPC:Ch, 2:1; DSPC:Ch:X, 4:1:1 where X=SA, DP or AML; and DSPC:Ch:AM, 8:3:1, were evaporated to dryness under nitrogen ($N_2$) and further dried under vacuum overnight. Each tube was filled with 0.6 ml phosphate 10 mM phosphate buffered 0.9 saline, pH 7.4 (PBS), containing 1mM nitrilotriacetic acid (NTA) and sonicated under $N_2$, for 5 to 15 minutes with an MSE brand probe sonicator equipped with a titanium microtip. Sonication yielded the small, unilamellar vesicles or vesicles used throughout these experiments.

Vesicles were annealed at 60° C for 10 minutes and centrifuged at 300 x g. Vesicles were separated from unencapsulated NTA with a 30 x 1.5 cm Sephadex G-50 column. Vesicle size was determined by electron microscopy of preparations. negatively stained with uranyl acetate. All vesicle types were shown by electron microscopy to have a mean diameter less than 0.1 μm (100 nm). For example, DSPC:Ch vesicles had a mean diameter of approximately 52.8nm. However, vesicles as large as approximately 200nm are believed to be satisfactory in obtaining the desired results of this invention.

The vesicles obtained as described above are chemically pure. By "chemically pure" is meant that the materials which constitute phospholipid vesicles are more than 98% pure. For example, when the phospholipid chemical added is distearoyl phosphatidylcholine, this material is used at more than 98% purity. The same constraint holds for other components, such as cholesterol, which compose the vesicle. The phospholipid vesicles obtained as described above are stable when injected into experimental animals.

The saccharide portions of aminomannose and aminomannitol derivatives of cholesterol extend externally from the phospholipid vesicles. Thus, when such derivatives are incorporated or associated into the bilayer of vesicles or other micelles, an amine moiety is provided that extends approximately 0.5-2.5nm preferably about 1nm, beyond the surface of the micelles. In the case of vesicles, it appears that the appropriate molecular design comprises a hydrophobic portion which serves to anchor the molecule within the vesicular bilayer, and a linking portion which is at least mildly hydrophilic which spans the requisite distance between the hydrophobic region and the amino functional group. The hydrophilicity is apparently required to prevent the link from internalizing within the bilayer also and thus serves to "extend" the amine from the surface. An example of a suitable extended amine within the context of this invention is a 6-aminomannose cholesterol derivative such as, for example, 6-(5-cholesten-3-β-yloxy) hexyl-6-amino-6-deoxyl-thio-D-mannopyranoside. In this example, the cholesterol portion provides the hydrophobic moiety, while the aminomannose is relatively hydrophilic. Other embodiments are also possible: other amino sugars attached to other cholesterol derivatives, for example, are equally suitable as alternative embodiments of the hydrophilic and hydrophobic portions. Polyamines and polyamino acids which can be bound covalently or associated by other means to the vesicle or other micelle surface may also be used. These materials and cholesterol tend to impart stability to the phospholipid vesicles. Cholesterol may also be included in the range of approximately 0% to 50% of cholesterol by weight of total phospholipid, the remainder constituting the phospholipids.

The chemically pure vesicle compositions discussed above are quite stable to leakage in vitro and in vivo. Phospholipid mixtures such as egg lecithin form more fluid membranes than pure phospholipids; as a result, vesicles from natural lecithin mixtures are less stable and are more likely to leak their contents than pure phospholipids.

In-111 Loading Procedure.

Loading of In-111 into preformed vesicles was facilitated by the presence of the ionophore A23187 in the lipid bilayer. In-111 was loaded into vesicles at 60°-80°C as described by Mauk and Gamble, Anal. Bioc., 94, 302-307 (1979). Incubations were terminated by the addition of 0.1 ml of 10mM EDTA in phosphate buffered 0.9% sodium chloride, pH 7.4 (PBS), and unencapsulated In-111 was separated from the loaded vesicles by chromatography on Sephadex G-50. Up to 90% of the added In-111 could be incorporated into preformed vesicles by this technique, and specific activities of up to 300 microcuries (μCi)/mg lipid have been obtained.

EMT6 Tumor Model.

Male BALB/c mice weighing 20-25 g were injected subcutaneously on the right hind leg with 5 x $10^5$ EMT6 cells in 0.1 ml sterile phosphate buffered saline. Tumors were allowed to grow for 10-20 days prior to using these animals for imaging studies. At this stage, tumors weighed between 0.2 and 0.4 g. Up to 0.5 ml of PBS containing 1-2 mg vesicles loaded with up to 30 μCi In-111 were injected into the tail vein of each animal. Control animals were injected with In-111-NTA which had not been encapsulated in vesicles.

Gamma Camera Imaging.

At one (1) hour and at twenty-four (24) hours after injecting In-111 loaded vesicles, each animal was anesthetized with 40 mg/kg sodium pentobarbital and positioned on a platform 12 cm from the gamma scintillation camera equipment with a 6 mm pinhold. Whole-body dorsal images were acquired on x-ray film and corresponding digitized data were stored on magnetic discs for computer analysis.

Biodistribution of Radioactivity.

At twenty-four (24) hours, animals were sacrificed and dissected to determine the organ distribution of radioactivity. Organs or tissues were excised, washed in PBS, blotted dry and weighed. Radioactivity was measured in a well-type gamma-ray spectrometer and quantitated based on activity present in vesicles before injection. In some experiments, the gamma-ray perturbed angular correlation (PAC) spectroscopy technique was used to measure the rotational correlation time of the In-111 in individual tissues and thereby assess the proportion of isotope remaining in intact vesicles. (Mauk and Gamble, P.N.A.S. (USA), 76, p. 765-769 (1979)),

Autoradiography

For autoradiography small, neutral, unilamellar vesicles were prepared with a composition similar to that described above for the In-111 labeling, except that [H-3]-dipalmitoyl phosphatidylcholine (H-3]-DPPC) was added as a marker and to produce autoradiographic exposure.

For autoradiographic studies, EMT6 tumor fragments (25-50 mg) were implanted subcutaneously in female BALB/C mice 5 to 12 days prior to the experiment. EMT6 tumor bearing mice were then given intravenous injections of 225-350 μCi of [H-3]-labeled vesicles, or normal saline as a background control. Fifteen hours later, the animals were sacrificed. Tumor, heart, skeletal muscle, liver, spleen and skin samples were removed, immediately immersed in the solution of 2% glutaraldehyde-2% paraformaldehyde, and sectioned into 1-2 mm pieces. The samples were further fixed in 1% osmium tetroxide, and then dehydrated and embedded in EPON® for thin sectioning.

Thin (1.5 $\mu$m) tissue sections were placed on microscope slides and coated with Ilford L4 photographic emulsion. Emulsions were exposed for 14-21 days and then developed. Tissues were lightly counterstained with 1% toluidine blue, and photomicrographs were taken.

Results

Whole body scintographs were made of tumor bearing mice which had been injected-intravenously with small In-111-NTA phospholipid vesicles 24 hours previously. EMT6 tumor images were clearly discernible in animals injected with neutral, negative and positively charged phospholipid vesicles.

The quality of tumor imaging is enhanced significantly using a vesicle blockade. In particular, neutral DSPC:Ch phospholipid vesicles deliver In-111 to EMT6 murine tumors in sufficient quantity to allow definitive localization of tumors by gamma camera imaging.

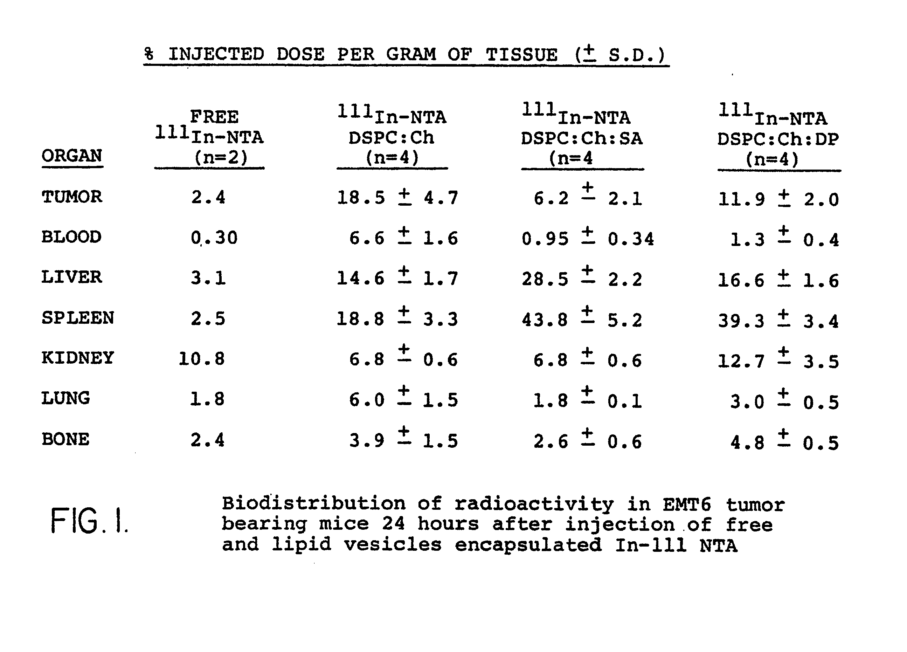

A comparison of the biodistribution of In-111-NTA delivery by each of these vesicle types can be made from the data presented in Figure 1. As will be seen from the second column of Figure 1, neutral phospholipid vesicles provided the best delivery of In-111 to tumor tissue. The specific targeting of the phospholipid vesicles to the tumors in this instance was at least as high as the targeting of the phospholipid vesicles to the liver or spleen, the usual target tissues of vesicles, and was nearly 8 times greater than the specific activity observed at the tumors when free In-111 NTA was injected in vivo. This has not been previously observed by others employing vesicles as tumor imaging agents. This will be seen from a comparison of the results shown in the first and second columns of Figure 1. It can also be seen in Figure 1 that, as liver and spleen uptake of In-111 decreases, the concentration of the phospholipid vesicles remaining in the blood increases. Also the increase in tumor associated radioactivity correlates approximately with the blood level of In-111.

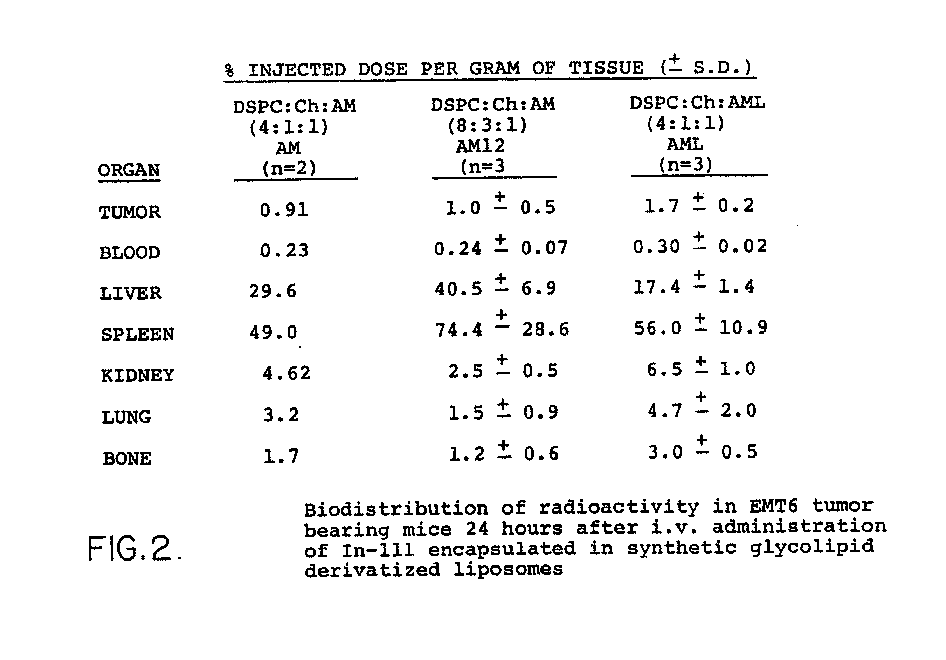

Applicants have previously demonstrated a strong association with EMT6 tumor cells in vitro of vesicles with the 6-aminomannose derivative of cholesterol. Applicants accordingly attempted tumor imaging with phospholipid vesicles of aminomannose derivatives of cholesterol where such vesicles were labeled with In-111. Applicant's observations in this experiment confirmed that the vast majority of In-111 in such phospholipid vesicles ultimately is deposited in the liver and spleen. Tumor images could not be obtained with such phospholipid vesicles as demonstrated in columns 2 and 3 of Figure 2 by the low deposition of radioactivity in the tumor. The low deposition of radioactivity in the tumor may result from the fact that most of such vesicles are taken up by the liver and spleen.

Vesicles with a lower concentration of the 6-AM derivative of cholesterol do not get trapped in the lung, so it seemed reasonable to assume that AM/2 vesicles (third column of Figure 2) loaded with In-111 might be better tumor imaging agents than the material shown in the second column of Figure 2. A comparison of the second and third columns of Figure 2 shows that this was not the case. In fact, the AM/2 vesicles had a very high affinity for the liver and spleen. For example, after a period of 24 hours from the time of the injection of the lipid vesicles in the blood stream, the combined radioactivity in the liver and spleen averaged greater than 75% of the total injected dose. This was the highest amount of liver and spleen uptake of vesicles observed of the several lipid composition studied.

Applicants have previously shown that positively charged vesicles were bound to EMT6 cells in vitro to a much greater extent than either, neutral or negatively charged vesicles. Applicants accordingly investigated AML derivatives of cholesterol, another synthetic glycolipid derivative with positive charge. These AML vesicles did show a lower affinity for liver and spleen (column 3 of Figure 2) and a slightly increased uptake by tumor compared to that provided by AM/2 vesicles (column 2 of Figure 2). However, this level of tumor-associated radioactivity was still three to ten times less than observed in the experiments with neutral, positive and negative vesicles as shown in Figure 1.

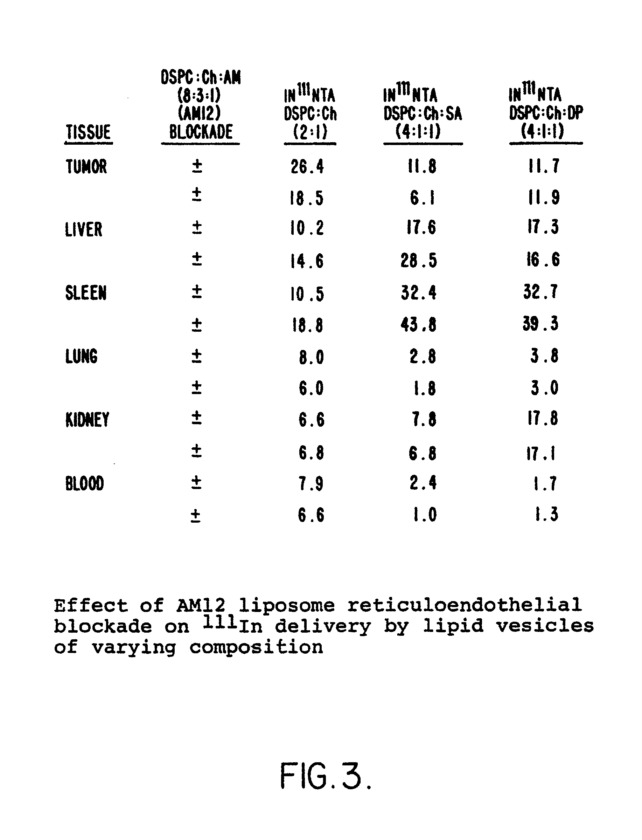

In further experiments, mice were injected with either a saline solution (minus, "-", blockade) or with 8 mg AM/2 vesicles one hour prior to injecting In-111 labeled vesicles of the compositions described in Figure 3. Tissue biodistribution was determined at twenty-four (24) hours as described above.

The saline solution provided a control and did not block the reticuloendothelial cells in the liver and spleen in the manner discussed above. The AM/2 vesicles provided a positive charge and were effective in blocking the reticuloendothelial cells in the liver and spleen. Since the reticuloendothelial cells in the liver and spleen were at least partially blocked with the AM/2 vesicles, any subsequent injection of phospholipid vesicles into the blood stream of the body had an increased opportunity for uptake by the tumor.

Figure 1 indicates the amount of In-111 targeted to the different parts of the body when phospholipid vesicles containing In-111 are introduced into the blood stream without any previous blockade of the reticuloendothelial cells in the liver and spleen. In contrast, Figure 3 indicates the amount of In-111 targeted to the different parts of the body when phospholipid vesicles containing In-111 are introduced into the blood stream after a previous blockade of the reticuloendothelial cells in the liver and spleen. As can be seen by a comparison of Figures 1 and 3, the amount of the In-111 targeted to the tumor was significantly increased in most instances using the blockade. Furthermore, the amount of the In-111 received at the liver and spleen using the blockade was significantly reduced from the amount of the In-111 received at the liver and spleen in Figure 1.

In the experiments discussed above, the second group of phospholipid vesicles to the be targeted to the tumor were introduced into the blood stream approximately one hour after the introduction of the initial group of phospholipid vesicles into the blood stream to block the reticuloendothelial cells in the liver and spleen. It will be appreciated that other time periods than one hour may also be used. For example, the time period may be considerably shorter than one hour. Since the phospholipid vesicles blocking the liver and spleen are effective for an extended period, the introduction of the phospholipid vesicles to target the tumor may be considered as concurrent with the introduction of the phospholipid vesicles to block the liver and spleen.

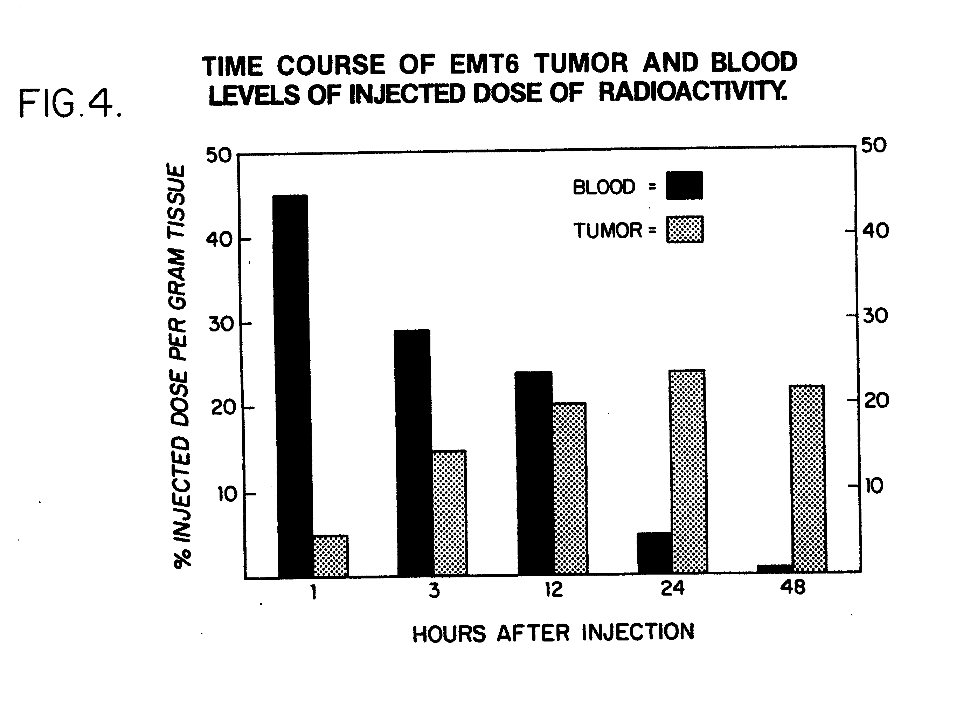

Figure 4 indicates tumor and blood radioactivity levels at various time points after injection of neutral vesicle (DSPC:Ch, 2:1 mole ratio)-encapsulated In-111 NTA. Tumor-associated radioactivity is maximal 24 hours after injection. Ninety percent of the radioactivity was cleared from the blood in 24 hours. This data suggests that Indium-associated vesicles circulate intact in the blood and are selectively accumulated in the tumor over time.

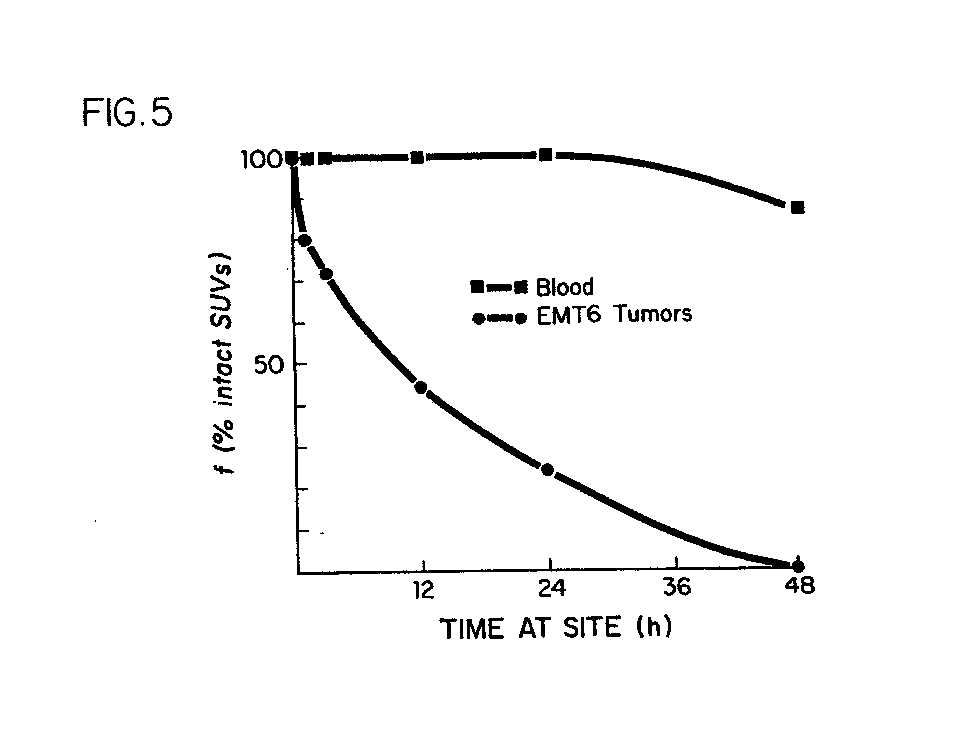

Gamma ray perturbed angular correlation (PAC) spectroscopy studies were performed on selected tissues at various times to confirm that the vesicles were intact in the bloodstream. Individual tumors and blood samples were examined by gamma-ray PAC spectrometry at 1 to 48 hours after injection of Indium-111 labeled phospholipid vesicles. The PAC results presented in Figure 5 as fractional intact vesicles show that over 80% of the blood-born radioactivity remains within vesicles up to 48 hours. On the other hand, the radioactivity accumulated in tumor is largely released from vesicles. This result indicates that the vesicles which become associated with tumors are being broken down or lysed and that the Indium-111 is binding to macromolecules such as proteins, whereas vesicles in the blood are remaining intact.

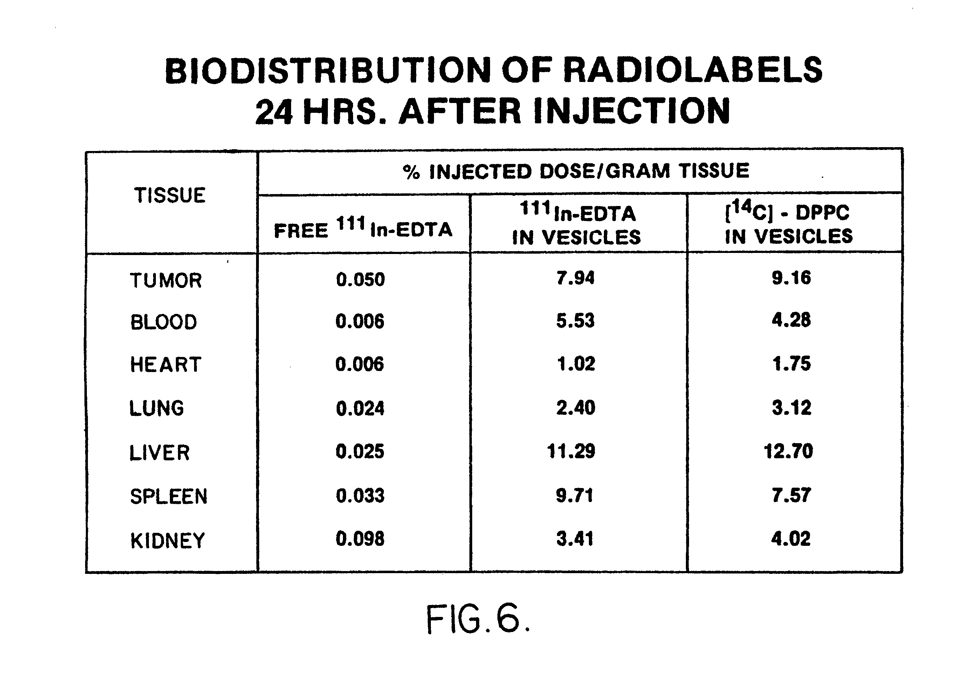

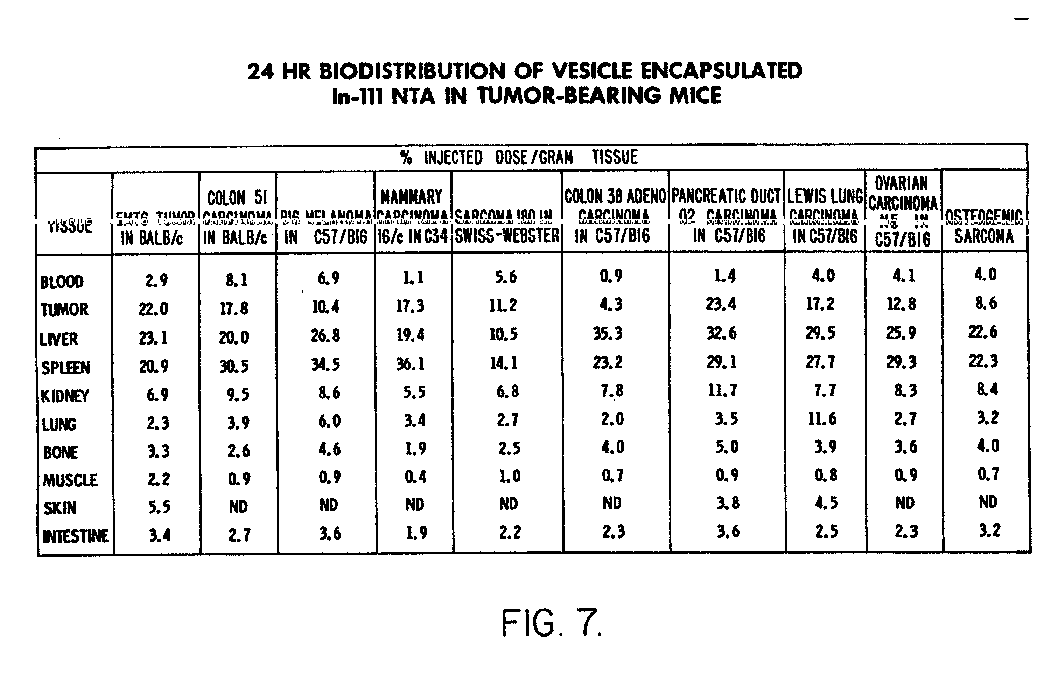

The biodistributions for free In-111-EDTA and vesicle-encapsulated In-111-EDTA were also studied to further demonstrate tumor localization of intact vesicles. (Columns 1 and 2, Figure 6). EDTA is a strong chelator as compared to NTA, and will not release Indium-111 to be bound by proteins. Furthermore, unbound Indium-111 EDTA is rapidly cleared from the blood and excreted via the kidneys. Thus, radioactivity remaining in the animal 24 hours after injection must come from or still be within intact vesicles and not from a protein bound intermediate. The results depicted in Figure 7 show that at twenty-four (24) hours encapsulated Indium-111 EDTA exhibits pharmacokinetics identical to C-14 labeled vesicles. Free In-111 EDTA however, does not accumulate significantly in any of the tissues because it is rapidly excreted.

The effect of labeling the membrane component rather than the internal aqueous space of the vesicles was then examined. Carbon 14-labeled DPPC tracer was added to vesicles in order to follow the biodistribution of the membrane component of the vesicles. Blood clearance and biodistributions within tissues were found to be similar to the experiments using In-111 labeling (Column 3 of Figure 6).

Biodistribution studies of vesicle-encapsulated Indium-111-NTA in mice bearing tumors of various types were also conducted. (Figure 7). Tumor associated radioactivity was at least 50% of the liver uptake on a per gram of tissue basis in seven of these tumor types. Lower relative tumor uptake was observed with colon adenocarcinoma 38; B-16 melanoma, and osteogenic sarcoma. These results suggest that different tumor types will accumulate Indium-111 to varying degrees.

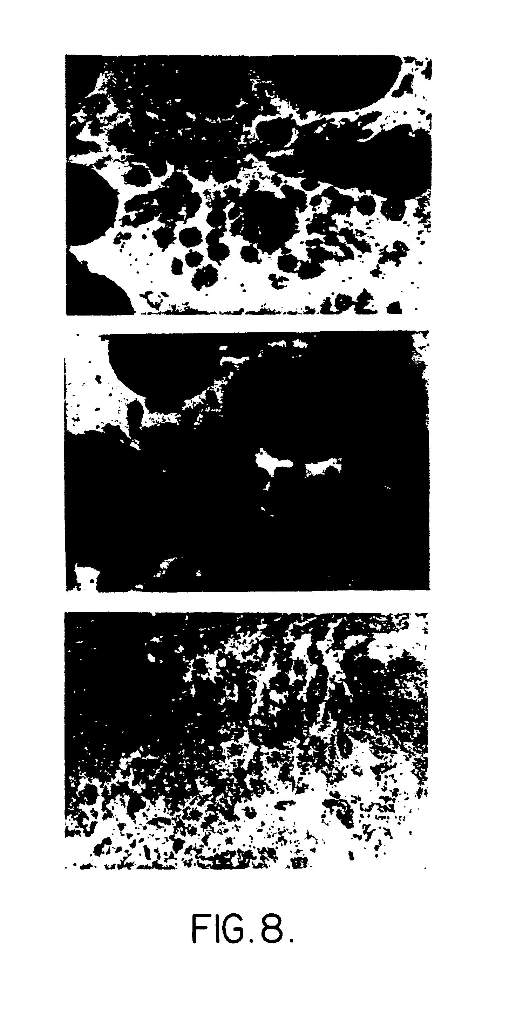

Autoradiography studies (Fig. 8) were performed using techniques that allow sectioning through human cells. Many grains of exposure directly over the dense layer of rapidly growing EMT6 tumor cells at or near the surface of the tumor indicate the presence of vesicles in the tumor cells. In contrast, the inner necrotic core of the tumor produced little autoradiographic exposure. Adipocytes and connective tissue at the periphery of the tumor mass also showed few grains of exposure.

Liver sections also showed high autoradiographic exposure as would be expected from the overall uptake of label. The uniform density of silver grains over the entire section of liver confirm that small vesicles are reaching hepatocytes, as has been reported by others. (Roerdink, et al., Bioc. et Biophys. Acta, 770, p. 195-202 (1984)). Spleen sections also showed much exposure as expected, but no distinct cell type could be identified as being responsible for the high spleenic labeling. Other tissues that were examined did not produce significant autoradiographic exposure.

Control tissue sections showed uniformly low photographic exposure, thus assuring that the experimental exposures obtained were not artifactual.

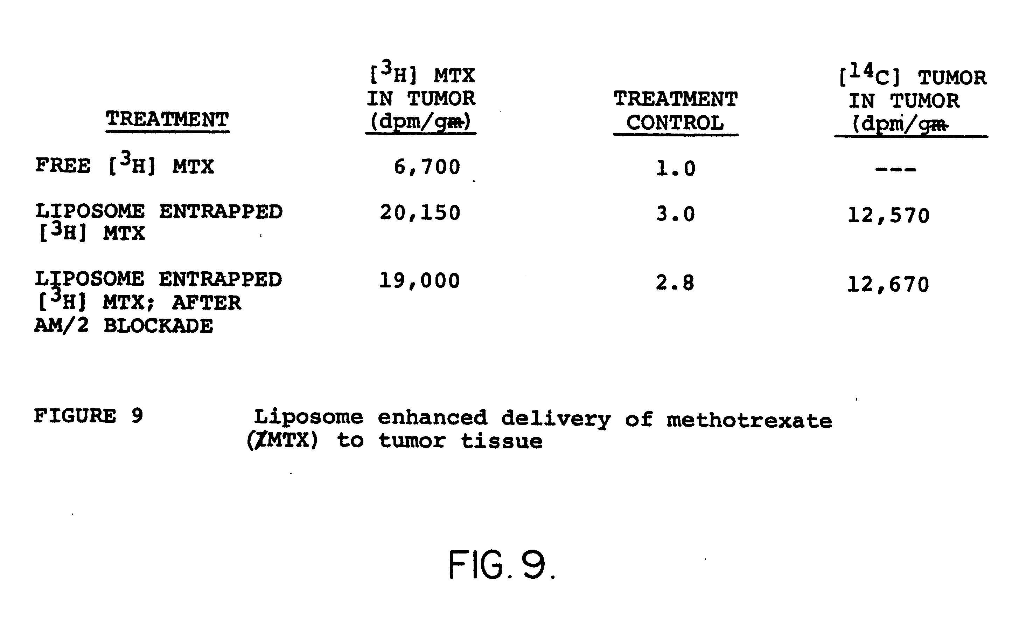

The phospholipid vesicles as constructed herein may be used to provide an enhanced delivery of drugs such as the chemotherapeutic agent methotrexate (MTX) to tumors in the body. This may be seen from the results of experiments specified in the table constituting Figure 9. To demonstrate MTX delivery, [H-3]methotrexate [H-3] (MTX) free and vesicle encapsulated, was injected directly into tumor-bearing mice, The amount of the [H-3] MTX in tumors was measured by scintillation counting after four hours. Phospholipid vesicles containing DSPC:Ch:SA in the ratio of 4:1:1 were labeled with [C-14] cholesterol oleate and [H-3] MTX was entrapped in the phospholipid vesicles. As will be seen, the amount of the phospholipid vesicles targeted to the tumors is almost three times greater than the amount of the free MTX directed to the tumor.

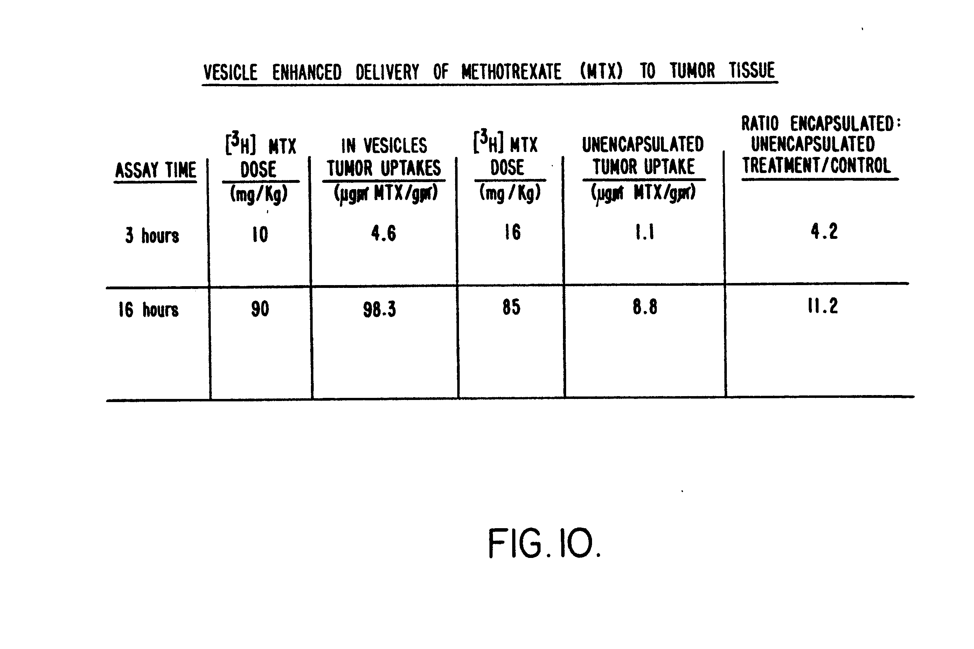

The MTX uptake by tumor was further enhanced using neutral vesicles of the preferred composition (DSPC:Ch in the mole ratio of 2:1). (Figure 10), The ratios of [H-3] MTX uptake, free vs. unencapsulated, were 4.2 and 11.2 fold at three hours and sixteen hours respectively.

There are several improvements in vesicle technology which are utilized in the present invention which may explain why better tumor imaging and delivery of intact vesicles into tumor cells are achieved. One such improvement is the use of small, chemically pure phospholipid containing hydrocarbon chains of at least 18 carbon atoms provides vesicles that are stable in vivo and are thus capable of delivering the desired imaging agent or chemotherapeutic agent to a tumor.

## Claims

1. The use of a micellular particle composition, said particles having a chemotherapeutic agent encapsulated therein and comprising a chemically pure (>98% pure)phospholipid and zero to fifty percent cholesterol, by weight of phospholipid, said particles having a size less than 200 nm, for the preparation of an intravenously administrable pharmaceutical composition useful for the delivery of said particle composition and chemotherapeutic agent into tumor cells.

2. The use according to claim 1 wherein said phospholipid is distearoylphosphatidylcholine.

3. The use according to claims 1 or 2 wherein, when methotrexate is said chemotherapeutic agent, said particles are capable of accumulating in tumor cells in quantities of three times or greater than free methotrexate.

4. The use according to claim 3 wherein said accumulation in tumor cells is at least four times greater than free chemotherapeutic agent at three hours following introduction into the bloodstream and at least 11 times greater at 16 hours following introduction into the bloodstream.

5. The use according to claims 1 to 4 wherein the administration of said particles is preceded by the intravenous use of micellular particles of less than 200 nm, comprising chemically pure- (>98% pure) phospholipid molecules having positively charged molecules extending externally from the particles incorporated therewith, said micellular particles to be introduced into the bloodstream of a body to block macrophages in the body.

6. The use according to claims 1 through 5 wherein said micellular particles having a chemotherapeutic agent encapsulated therein are uncharged particles.

7. The use according to claims 1 through 6 wherein said micellular particles are in the form of spherical unilamellar phospholipid vesicles.

## Revendications

1. Utilisation d'une composition de particules micellaires dans lesquelles est encapsulé un agent chimiothérapeutique et qui comprennent un phospholipide chimiquement pur (pureté supérieure à 98 %) et zéro à cinquante pour cent de cholestérol par rapport au poids de phospholipide, ces particules ayant une taille inférieure à 200 nm, pour la préparation d'une composition pharmaceutique administrable par voie intraveineuse utilisable pour la délivrance de cette composition de particules et d'un agent chimiothérapeutique dans des cellules tumorales.

2. Utilisation selon la revendication 1, dans laquelle ce phospholipide est la distéaroylphosphatidylcholine.

3. Utilisation selon les revendications 1 ou 2, dans laquelle lorsque du méthotrexate est cet agent chimiothérapeutique, ces particules sont capables de s'accumuler dans les cellules tumorales dans des quantités trois fois plus élevées ou davantage que le méthotrexate libre.

4. Utilisation selon la revendication 3, dans laquelle cette accumulation dans les cellules tumorales est au moins quatre fois supérieure à celle de l'agent chimiothérapeutique libre trois heures après l'introduction dans le courant sanguin et au moins onze fois supérieure seize heures après l'introduction dans le courant sanguin.

5. Utilisation selon les revendications 1 à 4, dans laquelle l'administration de ces particules est précédée par l'utilisation intraveineuse de particules micellaires de moins de 200 nm, comprenant des molécules de phospholipide chimiquement pures (pureté supérieure à 98 %) ayant des molécules positivement chargées s'étendant vers l'extérieur à partir des particules avec lesquelles elles sont incorporées, ces particules micellaires devant être introduites dans le courant sanguin d'un organisme pour bloquer les macrophages dans l'organisme.

6. Utilisation selon les revendications 1 à 5, dans laquelle ces particules micellaires dans lesquelles est encapsulé un agent chimiothérapique sont des particules non chargées.

7. Utilisation selon les revendications 1 à 6, dans laquelle ces particules micellaires sont sous la forme de vésicules de phospholipides unilamellaires sphériques.

## Ansprüche

1. Verwendung einer Mizellteilchen-Zusammensetzung, in der die Teilchen darin eingekapselt eine chemotherapeutische Substanz enthalten, und ein chemisch reines (> 98% rein) Phospholipid und 0 bis 50 % Cholesterin, bezogen auf das Gewicht des Phospholipids, umfassen, und die Teilchen eine Größe von < 200 nm besitzen, zur Herstellung einer intravenös verabreichbaren pharmazeutischen Zusammensetzung, die geeignet ist zum Transport dieser Teilchen und der chemotherapeutischen Substanz in Tumorzellen.

2. Verwendung nach Anspruch 1, dadurch gekennzeichnet, daß das Phospholipid Distearoylphosphatidylcholin ist.

3. Verwendung nach Anspruch 1 oder 2, dadurch gekennzeichnet, daß, wenn Methotrexat die chemotherapeutische Substanz ist, die Teilchen dazu fähig sind, sich in Tumorzellen in dreifachen oder größeren Mengen als freies Methotrexat anzureichen.

4. Verwendung nach Anspruch 3, dadurch gekennzeichnet, daß die Anreicherung in Tumorzellen 3 Stunden nach der Einführung in den Blutstrom mindestens 4 mal größer ist als mit der freien chemotherapeutischen Substanz, und 16 Stunden nach der Einführung in den Blutstrom mindestens 11 mal größer ist.

5. Verwendung nach den Ansprüchen 1 bis 4, dadurch gekennzeichnet, daß der Verabreichung der Teilchen eine intravenöse Verwendung von Mizellteilchen von < 200 nm vorausgeht, die chemisch reine (> 98 % rein) Phospholipidmoleküle umfassen, die positiv geladene Moleküle besitzen, die sich von den eingeschlossenen Teilchen nach außen erstrecken, und die Mizellteilchen in den Blutstrom eines Körpers eingeführt werden, um Makrophagen im Körper zu blockieren.

6. Verwendung nach den Ansprüchen 1 bis 5, dadurch gekennzeichnet, daß die Mizellteilchen, die eine darin eingeschlossene chemotherapeutische Substanz enthalten, ungeladene Teilchen sind.

7. Verwendung nach den Ansprüchen 1 bis 6, dadurch gekennzeichnet, daß die Mizellteilchen in der Form sphärischer unilamellarer Phospholipidvesikel vorliegen.

% INJECTED DOSE PER GRAM OF TISSUE (± S.D.)

| ORGAN | FREE $^{111}$In-NTA (n=2) | $^{111}$In-NTA DSPC:Ch (n=4) | $^{111}$In-NTA DSPC:Ch:SA (n=4 | $^{111}$In-NTA DSPC:Ch:DP (n=4) |
|---|---|---|---|---|
| TUMOR | 2.4 | 18.5 ± 4.7 | 6.2 ± 2.1 | 11.9 ± 2.0 |
| BLOOD | 0.30 | 6.6 ± 1.6 | 0.95 ± 0.34 | 1.3 ± 0.4 |
| LIVER | 3.1 | 14.6 ± 1.7 | 28.5 ± 2.2 | 16.6 ± 1.6 |
| SPLEEN | 2.5 | 18.8 ± 3.3 | 43.8 ± 5.2 | 39.3 ± 3.4 |
| KIDNEY | 10.8 | 6.8 ± 0.6 | 6.8 ± 0.6 | 12.7 ± 3.5 |
| LUNG | 1.8 | 6.0 ± 1.5 | 1.8 ± 0.1 | 3.0 ± 0.5 |
| BONE | 2.4 | 3.9 ± 1.5 | 2.6 ± 0.6 | 4.8 ± 0.5 |

FIG. 1. Biodistribution of radioactivity in EMT6 tumor bearing mice 24 hours after injection of free and lipid vesicles encapsulated In-111 NTA

| ORGAN | % INJECTED DOSE PER GRAM OF TISSUE (± S.D.) | | |
|---|---|---|---|
| | DSPC:Ch:AM (4:1:1) AM (n=2) | DSPC:Ch:AM (8:3:1) AM12 (n=3 | DSPC:Ch:AML (4:1:1) AML (n=3) |
| TUMOR | 0.91 | 1.0 ± 0.5 | 1.7 ± 0.2 |
| BLOOD | 0.23 | 0.24 ± 0.07 | 0.30 ± 0.02 |
| LIVER | 29.6 | 40.5 ± 6.9 | 17.4 ± 1.4 |
| SPLEEN | 49.0 | 74.4 ± 28.6 | 56.0 ± 10.9 |
| KIDNEY | 4.62 | 2.5 ± 0.5 | 6.5 ± 1.0 |
| LUNG | 3.2 | 1.5 ± 0.9 | 4.7 ± 2.0 |
| BONE | 1.7 | 1.2 ± 0.6 | 3.0 ± 0.5 |

FIG. 2.

Biodistribution of radioactivity in EMT6 tumor bearing mice 24 hours after i.v. administration of In-111 encapsulated in synthetic glycolipid derivatized liposomes

| TISSUE | DSPC:Ch:AM (8:3:1) (AM12) BLOCKADE | $IN^{111}$NTA DSPC:Ch (2:1) | $IN^{111}$NTA DSPC:Ch:SA (4:1:1) | $IN^{111}$NTA DSPC:Ch:DP (4:1:1) |
|---|---|---|---|---|
| TUMOR | ± | 26.4 | 11.8 | 11.7 |
| | ± | 18.5 | 6.1 | 11.9 |
| LIVER | ± | 10.2 | 17.6 | 17.3 |
| | ± | 14.6 | 28.5 | 16.6 |
| SLEEN | ± | 10.5 | 32.4 | 32.7 |
| | ± | 18.8 | 43.8 | 39.3 |
| LUNG | ± | 8.0 | 2.8 | 3.8 |
| | ± | 6.0 | 1.8 | 3.0 |
| KIDNEY | ± | 6.6 | 7.8 | 17.8 |
| | ± | 6.8 | 6.8 | 17.1 |
| BLOOD | ± | 7.9 | 2.4 | 1.7 |
| | ± | 6.6 | 1.0 | 1.3 |

Effect of AM12 liposome reticuloendothelial blockade on $^{111}$In delivery by lipid vesicles of varying composition

FIG. 3.

FIG. 4.

TIME COURSE OF EMT6 TUMOR AND BLOOD LEVELS OF INJECTED DOSE OF RADIOACTIVITY.

BLOOD =
TUMOR =
% INJECTED DOSE PER GRAM TISSUE
50
40
30
20
10
1
3
12
24
48
HOURS AFTER INJECTION

f (% intact SUVs)
100
50
TIME AT SITE (h)
12
24
36
48
Blood
EMT6 Tumors

FIG. 5

# BIODISTRIBUTION OF RADIOLABELS 24 HRS. AFTER INJECTION

| TISSUE | % INJECTED DOSE/GRAM TISSUE | | |
|---|---|---|---|
| | FREE $^{111}$In-EDTA | $^{111}$In-EDTA IN VESICLES | [$^{14}$C] - DPPC IN VESICLES |
| TUMOR | 0.050 | 7.94 | 9.16 |
| BLOOD | 0.006 | 5.53 | 4.28 |
| HEART | 0.006 | 1.02 | 1.75 |
| LUNG | 0.024 | 2.40 | 3.12 |
| LIVER | 0.025 | 11.29 | 12.70 |
| SPLEEN | 0.033 | 9.71 | 7.57 |
| KIDNEY | 0.098 | 3.41 | 4.02 |

FIG.6.

## 24 HR BIODISTRIBUTION OF VESICLE ENCAPSULATED In-111 NTA IN TUMOR-BEARING MICE

| | % INJECTED DOSE/GRAM TISSUE | | | | | | | | | |
|---|---|---|---|---|---|---|---|---|---|---|
| TISSUE | EMT6 TUMOR IN BALB/c | COLON 51 CARCINOMA IN BALB/c | B16 MELANOMA IN C57/B16 | MAMMARY CARCINOMA 16/c IN C34 | SARCOMA 180 IN SWISS-WEBSTER | COLON 38 ADENO CARCINOMA IN C57/B16 | PANCREATIC DUCT 02 CARCINOMA IN C57/B16 | LEWIS LUNG CARCINOMA IN C57/B16 | OVARIAN CARCINOMA M5 IN C57/B16 | OSTEOGENIC SARCOMA |
| BLOOD | 2.9 | 8.1 | 6.9 | 1.1 | 5.6 | 0.9 | 1.4 | 4.0 | 4.1 | 4.0 |
| TUMOR | 22.0 | 17.8 | 10.4 | 17.3 | 11.2 | 4.3 | 23.4 | 17.2 | 12.8 | 8.6 |
| LIVER | 23.1 | 20.0 | 26.8 | 19.4 | 10.5 | 35.3 | 32.6 | 29.5 | 25.9 | 22.6 |
| SPLEEN | 20.9 | 30.5 | 34.5 | 36.1 | 14.1 | 23.2 | 29.1 | 27.7 | 29.3 | 22.3 |
| KIDNEY | 6.9 | 9.5 | 8.6 | 5.5 | 6.8 | 7.8 | 11.7 | 7.7 | 8.3 | 8.4 |
| LUNG | 2.3 | 3.9 | 6.0 | 3.4 | 2.7 | 2.0 | 3.5 | 11.6 | 2.7 | 3.2 |
| BONE | 3.3 | 2.6 | 4.6 | 1.9 | 2.5 | 4.0 | 5.0 | 3.9 | 3.6 | 4.0 |
| MUSCLE | 2.2 | 0.9 | 0.9 | 0.4 | 1.0 | 0.7 | 0.9 | 0.8 | 0.9 | 0.7 |
| SKIN | 5.5 | ND | ND | ND | ND | ND | 3.8 | 4.5 | ND | ND |
| INTESTINE | 3.4 | 2.7 | 3.6 | 1.9 | 2.2 | 2.3 | 3.6 | 2.5 | 2.3 | 3.2 |

FIG. 7.

FIG. 8.

| TREATMENT | $[^{3}H]$ MTX IN TUMOR (dpm/gm) | TREATMENT CONTROL | $[^{14}C]$ TUMOR IN TUMOR (dpm/gm |
|---|---|---|---|
| FREE $[^{3}H]$ MTX | 6,700 | 1.0 | --- |
| LIPOSOME ENTRAPPED $[^{3}H]$ MTX | 20,150 | 3.0 | 12,570 |
| LIPOSOME ENTRAPPED $[^{3}H]$ MTX; AFTER AM/2 BLOCKADE | 19,000 | 2.8 | 12,670 |

FIGURE 9 Liposome enhanced delivery of methotrexate (MTX) to tumor tissue

FIG. 9.

VESICLE ENHANCED DELIVERY OF METHOTREXATE (MTX) TO TUMOR TISSUE

| ASSAY TIME | [$^3$H] MTX DOSE (mg/Kg) | IN VESICLES TUMOR UPTAKES (μgm MTX/gm) | [$^3$H] MTX DOSE (mg/Kg) | UNENCAPSULATED TUMOR UPTAKE (μgm MTX/gm) | RATIO ENCAPSULATED: UNENCAPSULATED TREATMENT/CONTROL |
|---|---|---|---|---|---|
| 3 hours | 10 | 4.6 | 16 | 1.1 | 4.2 |
| 16 hours | 90 | 98.3 | 85 | 8.8 | 11.2 |

FIG. 10.